# EUROPEAN PATENT APPLICATION

(11) **EP 1 705 325 A2**
(43) Date of publication of application: **27.09.2006**
(21) Application number: 06001252.3
(22) Date of filing: 20.01.2006
(51) Int. Cl.: E05D 11/00

(54) **Communication device**

(30) Priority: 10.02.2005 JP 2005034284
(71) Applicant: SHARP KABUSHIKI KAISHA, Osaka-shi, Osaka 545-8522 (JP)
(72) Inventor: Yoshida, Hideo, Hiroshima-shi Hiroshima 734-0036 (JP); Inobe, Hiroyuki, Higashihiroshima-shi Hiroshima 739-0144 (JP); Kajihara, Sunao, Higashihiroshima-shi Hiroshima 739-0024 (JP)
(74) Representative: Müller - Hoffmann & Partner

(57) **Abstract**

A communication device comprising a first housing (3) having a first substrate (2) and a second housing (5) having a second substrate (4), wherein the first substrate (2) and the second substrate (4) are connected by a signal wire (10) including a plurality of thin electric wires (11), each of which is made of a conductor (11a) covered with an insulator (11b), the plurality of thin electric wires (11) forming the signal wire (10) are tied in a bundle at a center portion except the ends thereof and covered with a ground wire mesh (16) made of a plurality of conductive wires knitted into a mesh structure and the ends of the ground wire mesh (16) are connected to the first substrate (2) and the second substrate (4), respectively.

## Description

### BACKGROUND OF THE INVENTION

### 1. Field of the Invention

The present invention relates to a communication device including a first housing having a first substrate and a second housing having a second substrate.

### 2. Description of Related Art

Foldable communication devices including a first housing having a first substrate, a second housing having a second substrate and a flexible substrate for electrically connecting the first and second housings have conventionally been known as described in the pamphlet of published International Application No. 02/003665.

In recent years, however, various needs have arisen for the communication devices. For example, a liquid crystal display part provided in the first housing is required to be rotatable such that the liquid crystal display part is flipped over or the orientation of the liquid crystal display part is changed between portrait and landscape. In such cases, if signal wires are formed on the flexible substrate as described in the pamphlet of published International Application No. 02/003665, the signal wires cannot keep up with the movement of the liquid crystal display part because of lack of flexibility.

Further, there is also a need of eliminating a ground potential difference between the first and second substrates by connecting the substrates with a ground wire so that working error of the communication devices is eliminated. In order to eliminate the ground potential difference, it is simply necessary to provide a ground wire having a higher conduction capacity than the signal wires. If the above-described flexible substrate is replaced with a plurality of thin electric wires for greater flexibility of the signal wires, the diameter of a bundle of the wires becomes large in order to ensure the conduction capacity required for eliminating the ground potential difference. The bundle of the wires is difficult to arrange in the first and second housings and cannot be moved smoothly. As a result, the communication device cannot be smoothly opened or closed.

### SUMMARY OF THE INVENTION

In the light of the above-described problems, the present invention has been achieved. An object of the present invention is to obtain an easy-to-assemble communication device by adding a twist to the structure of the signal wires for connecting the first and second substrates in order to eliminate the ground potential difference between the substrates and reduce the working error.

In order to achieve the object, according to the present invention, a plurality of thin electric wires are tied in a bundle to function as a signal wire and the signal wire is covered with a ground wire mesh made of a plurality of conductive wires knitted into a mesh structure.

Specifically, the present invention is directed to a communication device including a first housing having a first substrate and a second housing having a second substrate. The first substrate and the second substrate are connected by a signal wire including a plurality of thin electric wires, each of which is made of a conductor covered with an insulator. The plurality of thin electric wires forming the signal wire are tied in a bundle at a center portion except the ends thereof and covered with a ground wire mesh made of a plurality of conductive wires knitted into a mesh structure. The ends of the ground wire mesh are connected to the first substrate and the second substrate, respectively.

According to the above-described structure, signal transmission between the first and second substrates is carried out through the thin electric wires, each of which is made of a conductor covered with an insulator. The ground wire mesh makes it possible to obtain enough capacity to eliminate the ground potential difference between the first and second substrates. Since the thin electric wires for forming the signal wire are tied in a bundle at the center portion except the ends thereof and covered with the ground wire mesh made of a plurality of conductive wires knitted into a mesh structure, the diameter of the obtained signal wire is kept at a minimum and the flexibility is obtained.

It is preferred that each of the conductive wires for forming the ground wire mesh is made of a resin thread covered with copper foil.

According to the above-described structure, the ground wire mesh is provided with high conductivity and flexibility.

It is preferred that the first housing and the second housing are connected by a hinge to be freely opened and closed by rotating on an open/close axis of the hinge, a through hole having the open/close axis as the center is formed in the hinge and the signal wire covered with the ground wire mesh passes through the through hole formed along the open/close axis.

According to the above-described structure, the signal wire covered with the ground wire mesh passes through the through hole which moves the least when the first and second housings are opened or closed. Therefore, the ground wire mesh is prevented from twisting or wearing away.

It is preferred that the first housing includes a base which is connected to the hinge and a liquid crystal display part which is supported on the base to be rotatable on a rotation shaft of the hinge extending perpendicular to the open/close axis such that the liquid crystal display part is flipped over, a through hole is formed in the center portion of the rotation shaft, the first substrate is attached to the liquid crystal display part and the signal wire covered with the ground wire mesh passes through the through hole formed in the rotation shaft of the base.

According to the above-described structure, even if a liquid crystal display part which is rotatable to be flipped over is provided in the first housing, the ground wire mesh is prevented from twisting or wearing away upon flipping over the liquid crystal display part because the signal wire passes through the through hole of the hinge formed along the rotation axis.

It is preferred that the hinge is made of metal, the first substrate and the second substrate are in contact with the hinge to be electrically conductive with each other and the ground wire mesh covering the signal wire is electrically conductive at a portion contacting the hinge.

According to the above-described structure, the ground wire mesh covering the signal wire is electrically conductive with the hinge which is electrically conductive with the first and second substrates. Therefore, the ground potential difference is reduced as much as possible with stability.

It is preferred that certain portions of the signal wire covered with the ground wire mesh are covered with marking tapes and the marking tapes function as markers for passing the signal wire through the hinge and connecting the signal wire to the first substrate and the second substrate.

According to the above-described structure, the signal wire covered with the ground wire mesh is arranged through the hinge as determined using the tapes as markers and connected to the first and second substrates. The tapes make the ground wire mesh made of a plurality of conductive wires hard to come undone. Further, if the marking tapes are bonded at portions corresponding to the through holes, the ground wire is prevented from wearing away.

### BRIEF DESCRIPTION OF THE DRAWINGS

FIG. **1** is an oblique general view illustrating a cellular phone according to an embodiment of the present invention.
FIG. **2** is an exploded oblique view illustrating a second substrate, a hinge and a signal wire.
FIG. **3** is an oblique view illustrating the second substrate and the hinge with the signal wire connected thereto.
FIG. **4** is a plan view illustrating a first substrate with the signal wire connected thereto.
FIG. **5** is a plan view illustrating the signal wire.
FIG. **6** is an enlarged sectional view illustrating electric wires for forming the signal wire.
FIG. **7** is a plan view illustrating connectors for the signal wire and the electric wires for forming the signal wire.
FIG. **8** is a plan view illustrating the electric wires for forming the signal wire covered with a fixing tape.
FIG. **9** is a plan view illustrating a ground wire mesh.
FIG. **10** is a sectional view taken along the line X-X indicated in FIG. 5.
FIG. **11** is a plan view illustrating how the signal wire is arranged.
FIG. **12** is an oblique view illustrating how the signal wire passes through the hinge.

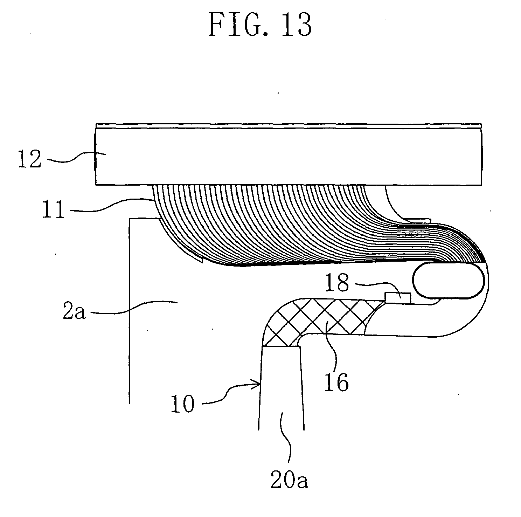

FIG. **13** is an enlarged view illustrating a first connector and the vicinity.

### DETAILED DESCRIPTION OF THE INVENTION

Hereinafter, an explanation of preferred embodiments of the present invention will be provided with reference to the drawings.

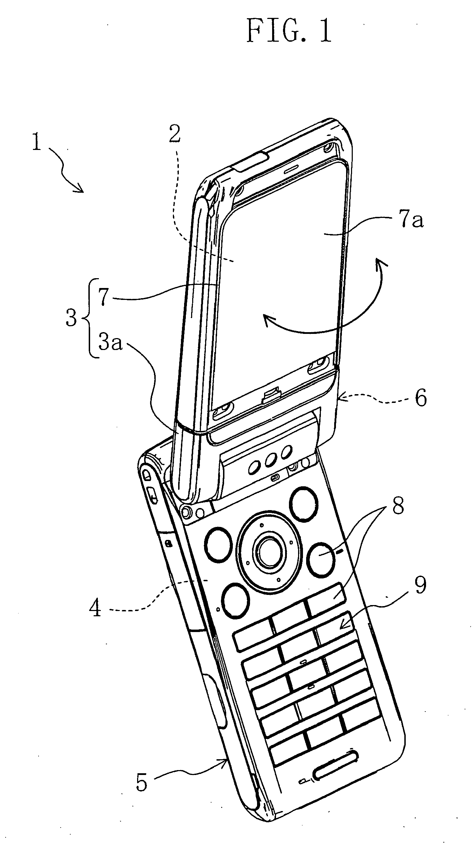

As shown in FIG. **1,** a cellular phone **1** as a communication device according to the present embodiment includes a first housing **3** having a first substrate **2** and a second housing **5** having a second substrate **4.** The first and second housings **3** and **5** are connected by a hinge **6** to be freely opened and closed.

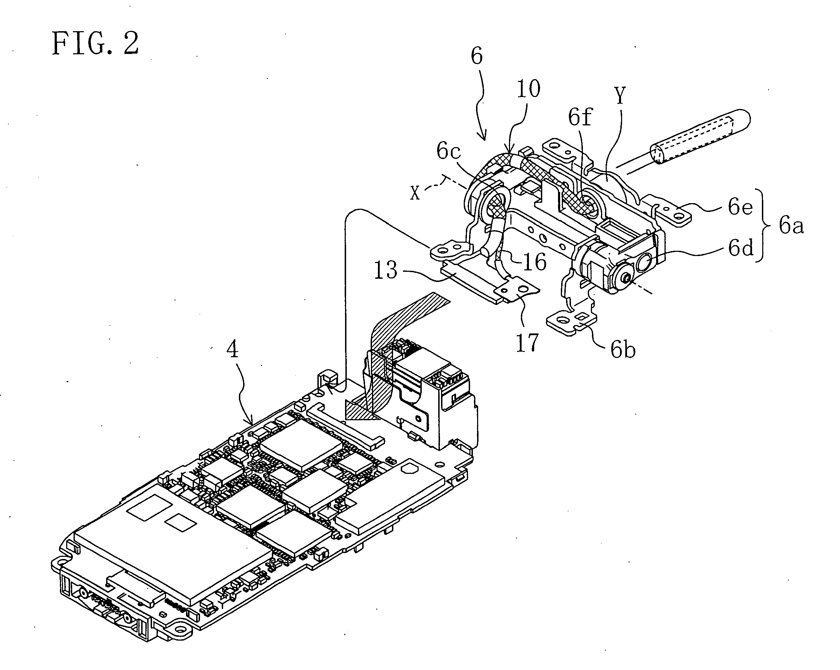

As shown in FIG. **2,** the hinge **6** is a rigid structure made of a combination of metallic components. The hinge **6** includes a first hinge part 6a facing the first housing **3** and a second hinge part **6b** facing the second housing **5.** The first and second hinge parts **6a** and **6b** are configured to be freely opened and closed by rotating on an open/close axis X extending in the horizontal direction. The hinge **6** also has a horizontal through hole **6c** having the open/close axis X as the center. A portion of the first hinge part **6a** near the open/close axis X is a first hinge body **6d** and a portion near a liquid crystal display part to be described later is an LCD (liquid crystal display) hinge part 6e which is supported to be rotatable on a rotation shaft Y extending perpendicular to the open/close axis X. A perpendicular through hole **6f is** formed in the center portion of the rotation shaft Y.

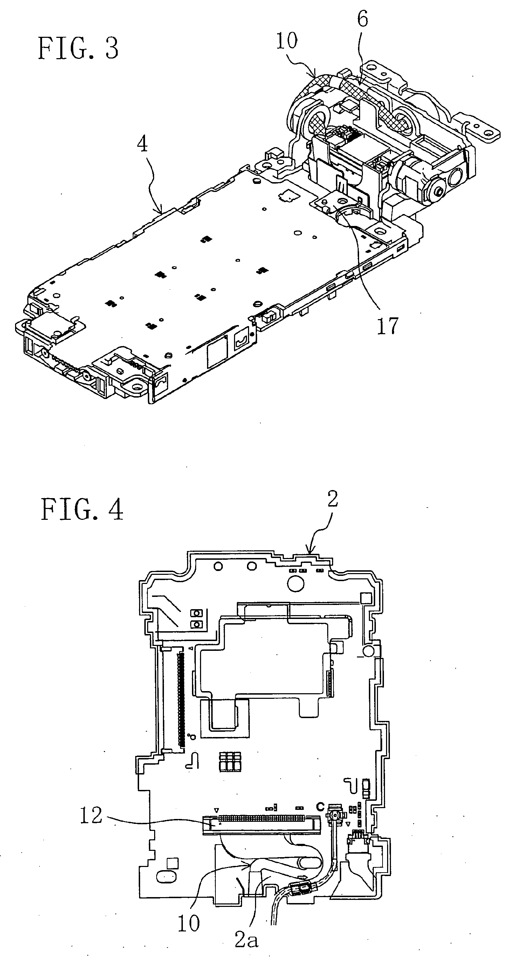

The first housing **3** includes a base **3a** to be connected to the hinge **6** and a liquid crystal display part **7** supported on the base **3a** to be rotatable on the rotation shaft Y such that the liquid crystal display part **7** is flipped over. Specifically, the first hinge body **6d** is installed in the base **3a** of the first housing 3 and the LCD hinge part **6e** is installed in the liquid crystal display part **7.** The liquid crystal display part **7** is provided with a rectangular liquid crystal display **7a.** The first substrate 2 is arranged behind the liquid crystal display **7a** as shown in FIG. **4.**

The second housing **5** is provided with an operation part **9** including a plurality of operation keys **8.** A user enjoys various functions of the cellular phone **1** by operating the operation keys **8.** As shown in FIGS. **2** and **3,** the second substrate **4** is provided behind the operation keys **8.**

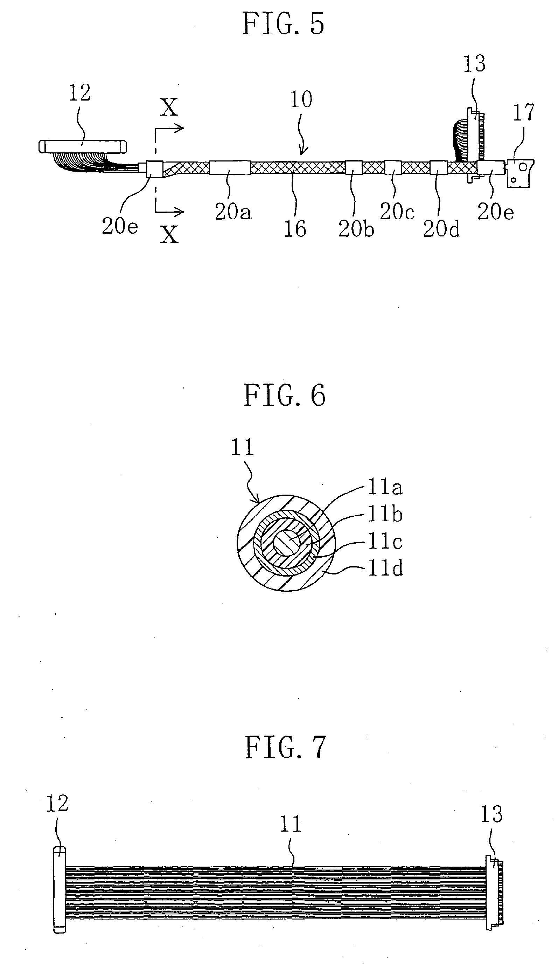

The first substrate **2** of the first housing **3** and the second substrate **4** of the second housing **5** are connected by a signal wire **10** (shown in FIG. **5)** including a plurality of thin electric wires **11.** As shown in an enlarged view of FIG. **6,** each of the electric wires **11** includes a conductor **11a** made of a silver-plated copper alloy wire, an insulator **11b** covering the conductor **11a,** a shield layer **11c** made of a tin-plated copper alloy wire covering the insulator **11b** and an outer sheath **11d** covering the shield layer **11c.**

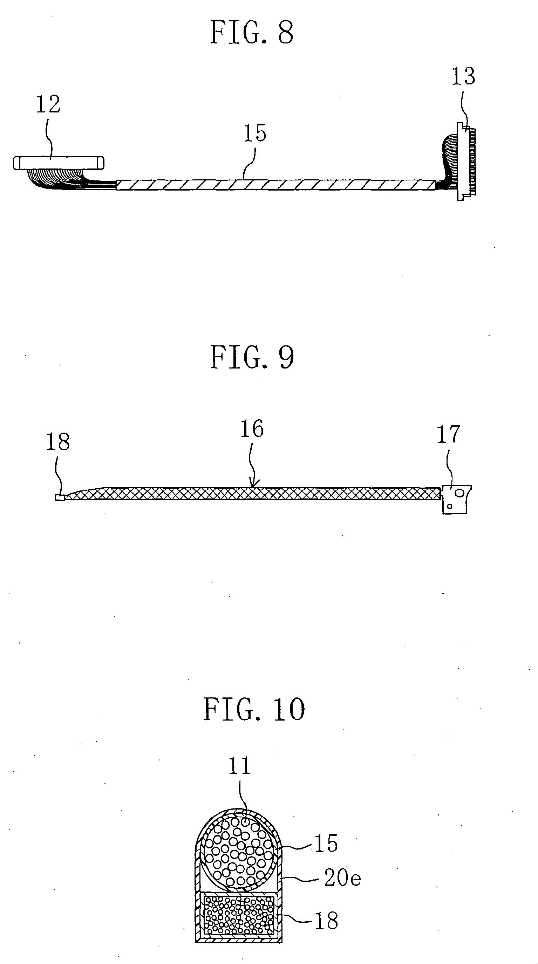

The ends of the electric wires **11** are connected to a first connecter **12** for the first housing 3 and a second connecter **13** for the second housing **5,** respectively, as shown in FIG. 7. As shown in FIG. **8,** the thin electric wires **11** are tied in a bundle at a center portion except portions near the first and second connectors **12** and **13** and covered with a resin-made fixing tape **15** to obtain the signal wire **10.**

The signal wire **10** is further covered with a ground wire mesh **16** which is made of a plurality of conductive wires knitted into a mesh structure. As shown in FIG. 9, a ground terminal 17 is connected to an end of the ground wire mesh 16 near the second connector 13. Further, as shown in FIG. 10, the other end of the signal wire 10 near the first connector 12 is crimped to provide a crimped part 18 such that the knitted conductive wires do not become undone. Though not shown, for example, each of the conductive wires for forming the ground wire mesh 16 is made of a 50-denier polyester thread covered with copper foil. Thus, the ground wire mesh 16 is provided with conductivity and flexibility. As shown in FIG. 3, the ground terminal 17 of the ground wire mesh 16 is connected to the second substrate 4 with screws (not shown). As shown in FIG. 4, the crimped part 18 is crimped onto an exposed part 2a of the first substrate 2.

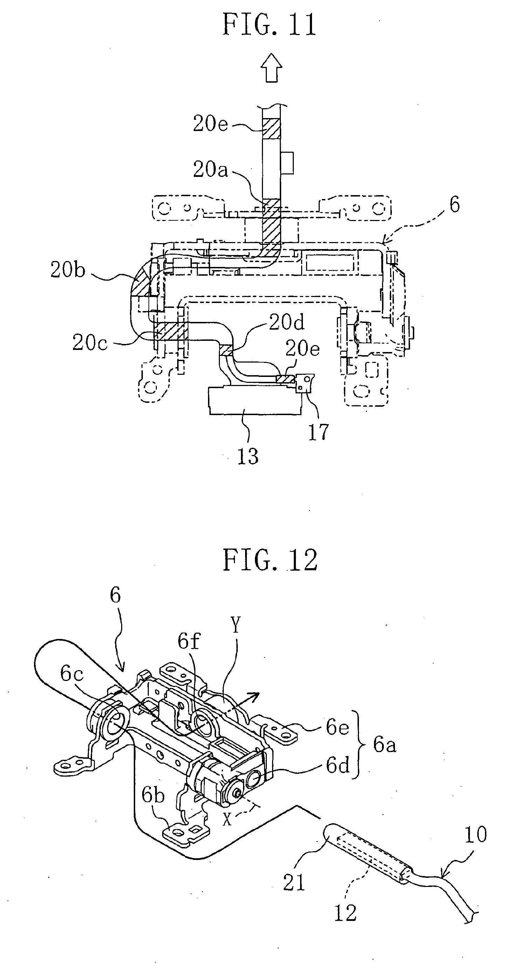

As shown in FIG. 5, certain portions of the signal wire 10 covered with the ground wire mesh 16 are covered with resin-made marking tapes 20a to 20e serving as markers that assist the placement of the signal wire 10 in the first housing 3, second housing 5 and hinge 6. For example, the marking tapes 20a to 20e of a certain length are bonded to the ground wire mesh **16** at six positions using an adhesive applied to the rear sides thereof. Specifically, as shown in FIG. 11, a first marking tape 20a is bonded at a position at a certain distance from the end of the ground wire mesh 16 near the first housing 3 and the portion covered with the marking tape 20a is arranged in the perpendicular through hole 6f of the base **3a.** A third marking tape **20c** is bonded at a position corresponding to the horizontal through hole **6c** of the hinge 6. A second marking tape **20b** is bonded at a position between the first and third marking tapes **20a** and **20c**. A fourth marking tape **20d** is bonded at a position between the third marking tape **20c** and the ground terminal 17. The both ends of the ground wire mesh 16 are also covered with the marking tapes 20e so that the conductive wires do not become undone.

Next, an explanation of how to arrange the signal wire in the cellular phone according to the present embodiment is provided.

First, the signal wire **10** as shown in FIG. **5** is prepared.

Then, as shown in FIG. **12,** the first connector **12** of the signal wire **10** is covered with a connector cover **21.** The connector cover **21** prevents the ground wire mesh **16** from coming undone and oil on the through holes **6c** and **6f** that may affect the signal transmission/reception from contacting the first connector **12.**

Then, the connector cover **21** is inserted into the horizontal through hole **6c** of the hinge **6** from the tip. At this time, the rear end of the connector cover **21** is pushed so that the signal wire **10** moves in a curve while attention is paid so as not to wrinkle the ground wire mesh **16.**

Then, the tip of the connector cover **21** is introduced into the perpendicular through hole **6f** of the base **3a** from the bottom.

Then, as shown in FIG. **11,** the signal wire **10** is pulled so that the marking tapes **20a** to **20d** come to the predetermined positions, and then the signal wire **10** is fixed.

Then, the second connector **13** of the signal wire **10** is connected to the second substrate **4** and the ground terminal **17** of the ground wire mesh **16** is connected to the second substrate **4.**

Then, finally, the connector cover **21** is detached and the first connector **12** is connected to the first substrate **2** with the crimped part **18** of the signal wire **10** faces upward and the ground wire mesh **16** is brought into contact with an exposed part **2a** of the first substrate **2.** At this time, the crimped part **18** of the ground wire mesh **16** is arranged to face upward (the direction opposite to the hinge **6).**

Thus, in the cellular phone **1** according to the present embodiment, a plurality of thin electric wires **11** for connecting the first and second substrates **2** and **4** are tied in a bundle at a center portion thereof and is covered with the ground wire mesh **16** made of a plurality of conductive wires knitted into a mesh structure, thereby providing the signal wire **10.** Both ends of the ground wire mesh **16** are connected to the first and second substrates **2** and **4,** respectively. As the ground wire mesh **16** having a large conduction capacity eliminates the ground potential difference between the first and second substrates **2** and **4,** signal transmission error is prevented. Further, the signal wire **10** becomes more flexible and the placement thereof becomes significantly easy.

According to the above-described embodiment, the ground wire mesh **16** is made of conductive resin wires covered with copper foil. As a result, the ground wire mesh **16** improves in conductivity and flexibility. Therefore, the cellular phone **1** becomes less likely to cause malfunction and easy to assemble.

According to the above-described embodiment, the first and second housings **3** and **5** are connected by the hinge **6** to be freely opened and closed by rotating on the open/close axis X. Further, the signal wire **10** covered with the ground wire mesh **16** passes through the horizontal through hole **6c** which moves the least when the first and second housings **3** and 5 are opened and closed. Therefore, the ground wire mesh **16** is prevented from twisting or wearing away, the open/close movement is not hindered by the signal wire **10** and the conductive wires are prevented from tearing away.

According to the above-described embodiment, the liquid crystal display part of the first housing **3** is supported to be rotatable on the rotational shaft such that the liquid crystal display part is flipped over and the signal wire **10** covered with the ground wire mesh **16** passes through the through hole formed in the rotational shaft. Therefore, the ground wire mesh **16** is prevented from twisting or wearing away upon flipping over the liquid crystal display part, the flip-over movement is not hindered by the signal wire **10** and the conductive wires are prevented from tearing away.

According to the above-described embodiment, the ground wire mesh **16** of the signal wire **10** is in contact with the hinge **6** which is electrically conductive with the first and second substrates **2** and **4** such that the ground potential difference is reduced as much as possible with stability. Therefore, the working error of the cellular phone **1** is prevented more effectively.

According to the above-described embodiment, certain portions of the signal wire **10** covered with the ground wire mesh **16** are covered with the marking tapes and the signal wire **10** is connected to the first and second substrates **2** and **4** using the tapes as markers. Therefore, the ground wire mesh **16** becomes hard to come undone and the signal wire **10** is arranged significantly easily.

### (Other embodiments)

The present invention may also have the following structures.

In the above-described embodiment, the present invention is directed to the folding cellular phone **1.** However, the present invention is also applicable to cellular phones which are not foldable. Further, the liquid crystal display part **7** may not be supported by the base **3a** of the first housing **3** to be rotated and flipped over. Even in such a case, the signal wire **10** according to the present invention is applicable.

In the above-described embodiment, the liquid crystal display part **7** of the first housing **3** includes the liquid crystal display **7a.** However, the liquid crystal display part **7** may include an organic electroluminescence display.
The above-described embodiment is given as an essentially preferable example and does not limit the present invention, the object of the present invention and the scope of application of the present invention.

## Claims

1. A communication device comprising a first housing (3) having a first substrate (2) and a second housing (5) having a second substrate (4), wherein
the first substrate (2) and the second substrate (4) are connected by a signal wire (10) including a plurality of thin electric wires (11), each of which is made of a conductor (11a) covered with an insulator (11b),
the plurality of thin electric wires (11) forming the signal wire (10) are tied in a bundle at a center portion except the ends thereof and covered with a ground wire mesh (16) made of a plurality of conductive wires knitted into a mesh structure and
the ends of the ground wire mesh (16) are connected to the first substrate (2) and the second substrate (4), respectively.

2. A communication device according to claim 1, wherein
each of the conductive wires for forming the ground wire mesh (16) is made of a resin thread covered with copper foil.

3. A communication device according to claim 1 or 2, wherein
the first housing (3) and the second housing (5) are connected by a hinge (6) to be freely opened and closed by rotating on an open/close axis (X) of the hinge (6),
a through hole (6c) having the open/close axis (X) as the center is formed in the hinge (6) and
the signal wire (10) covered with the ground wire mesh (16) passes through the through hole (6c) formed along the open/close axis (X).

4. A communication device according to claim 3, wherein
the first housing (3) includes a base which is connected to the hinge (6) and a liquid crystal display part (7) which is supported on the base to be rotatable on a rotation shaft (Y) of the hinge (6) extending perpendicular to the open/close axis (X) such that the liquid crystal display part (7) is flipped over,
a through hole (6f) is formed in the center portion of the rotation shaft (Y),
the first substrate (2) is attached to the liquid crystal display part (7) and
the signal wire (10) covered with the ground wire mesh (16) passes through the through hole formed in the rotation shaft (Y) of the base.

5. A communication device according to claim 3 or 4, wherein
the hinge (6) is made of metal,
the first substrate (2) and the second substrate (4) are in contact with the hinge (6) to be electrically conductive with each other and
the ground wire mesh (16) covering the signal wire (10) is electrically conductive at a portion contacting the hinge (6).

6. A communication device according to any one of claims 3 to 5, wherein
certain portions of the signal wire (10) covered with the ground wire mesh (16) are covered with marking tapes (20a, 20b, 20c, 20d) and
the marking tapes (20a, 20b, 20c, 20d) function as markers for passing the signal wire (10) through the hinge (6) and connecting the signal wire (10) to the first substrate (2) and the second substrate (4).
